# EUROPEAN PATENT SPECIFICATION

(11) **EP 3 744 251 B1**
(45) Date of publication and mention of the grant of the patent: **21.10.2026**
(21) Application number: 20176061.8
(22) Date of filing: 22.05.2020
(51) Int. Cl.: A61B 5/1455, A61B 5/0205, A61B 5/00, A61B 5/1491

(54) **PHOTOPLETHYSMOGRAPH DEVICE WITH THERMAL TRAPPING AND/OR WARMING CAPABILITY AND RELATED METHODS**
PHOTOPLETHYSMOGRAPHIEVORRICHTUNG MIT THERMISCHER WÄRMEEINFANGS- UND/ODER ERWÄRMUNGSFÄHIGKEIT UND ZUGEHÖRIGE VERFAHREN
DISPOSITIF DE PHOTOPLETHYSMOGRAPHIE AYANT DES CAPACITÉS DE PIÉGEAGE THERMIQUE ET/OU DE RÉCHAUFFEMENT ET PROCÉDÉS ASSOCIÉS

(30) Priority: 30.05.2019 US 201962854502 P; 13.06.2019 US 201962860862 P
(43) Date of publication of application: 02.12.2020
(73) Proprietor: Welch Allyn, Inc., Skaneateles Falls, NY 13153 (US)
(72) Inventor: BREMER, Edward C., Skaneateles Falls, NY New York 13153 (US)
(74) Representative: Reddie & Grose LLP

(56) References cited:
- US-A- 5 158 082
- US-A1- 2014 343 383
- US-A1- 2017 325 698
- MENDELSON Y ET AL: "SKIN REFLECTANCE OXIMETRY: IN VIVO MEASUREMENTS FROM THE FOREARM AND CALF", JOURNAL OF CLINICAL MONITORING, BOSTON, MA, US, vol. 7, 1 January 1991 (1991-01-01), pages 7 - 12, XP001040736, DOI: 10.1007/BF01617892

## Description

The subject matter described herein relates to photoplethsymography, and particularly to a photoplethsymography (PPG) device having thermal trapping and/or warming capability and associated methods of using the device.

Photoplethysmography (PPG) is a simple, low cost, optical technique used to detect volumetric changes in biological tissue. Applications of PPG include monitoring blood oxygen saturation, blood pressure, heart rate, respiration, and arterial compliance to name just a few *(Photoplethysmography (PPG),* https://www.news-medical.net/health/Photoplethysmography-(PPG).aspx, Susha Cheriyedath, M.Sc.).

In operation a conventional photoplethysmography device illuminates a tissue site alternately with light of first and second wavelengths, typically red (approximately 650 nm) and infrared (approximately 950 nm). Light which is not absorbed or otherwise dissipated penetrates through the tissue (transmittance PPG device) or is reflected back (reflectance PPG device). Either way the light arrives at a photodetector such as a photodiode. Taking blood oxygen saturation (SpO₂) as an example application, a processor estimates the SpO₂ value in a well-known manner as a function of the intensity of light received at the photodetector in response to the red illumination and the infrared illumination during both pulsatile and nonpulsatile phases of the patient's heart cycle.

MENDELSON, Y et al. Skin Reflectance Oximetry: In vivo measurements from the forearm and calf. Journal of clinical monitoring, Boston, MA, US, Vol. 7, 1 January 1991, pages 7-12, discloses a study that describes the results from a series of human experiments demonstrating the ability to measure arterial hemoglobin oxygen saturation (SaO2) from the forearm and calf using a reflectance pulse oximeter sensor. A special optical reflectance sensor that includes a heating element was interfaced to a temperature controller and a commercial Data-scope ACCUSAT pulse oximeter that was adapted for this study to perform as a reflectance pulse oximeter. The reflectance pulse oximeter sensor was evaluated in a group of 10 healthy adult volunteers during steady-state hypoxia. Hypoxia was induced by gradually lowering the inspired fraction of oxygen in the breathing gas mixture from 100 to 12%. Simultaneous SaO2 measurements obtained from the forearm and calf with two identical reflectance pulse oximeters were compared with SaO2 values measured by a finger sensor that was interfaced to a standard Datascope ACCUSAT transmittance pulse oximeter.

US5158082A1 discloses a method and apparatus for increasing the blood flow in a patient's tissue by warming the tissue with heat generated by a semiconductor device mounted in a sensor. The amount of beat generated is controlled by varying either the magnitude or the duty cycle of the current that flows through the semiconductor device, with the amount of heat required determined by either the temperature of the sensor's contact surface or the operating performance of the apparatus.

US 2014343383 A1 describes a measurement device including a light source which emits at least one kind of measurement light belonging to a predetermined wavelength band toward a measurement region formed of at least part of a living body, a detection unit in which a plurality of sensors are regularly arranged in a predetermined arrangement and which detect the measurement light emitted from the light source and passing through the living body with the plurality of sensors, and an analysis unit which performs analysis processing of specifying a measurement position for measuring information on pulsation along with activities of the living body from the measurement region based on a temporal change in an amount of light of the detected measurement light by use of a detection result detected by the detection unit.

One challenge associated with PPG devices is that the signal received at the photodetector contains both a static component and a fluctuating component (often referred to as DC and AC components). The DC component is attributable to things other than pulsatile arterial blood (e.g. flesh, bone) and is not typically of interest. The AC component is attributable to pulsating arterial blood flow and contains the information of interest. However the AC component is quite small in comparison to the DC component (on the order of 1% of the DC component). As a result, the signal may be difficult to detect and its information content may be difficult to extract.

Therefore, what is needed is a PPG device and associated methods that cause the AC component of the signal to be less difficult to detect and its information content easier to extract.

The present invention is defined by the independent claims. Preferred or optional features are set out in the dependent claims.

An apparatus, system, or method may comprise one or more of the following features.

According to a first aspect of the present disclosure, a photoplethysmography (PPG) device may include an equipment module which, in turn, may include a first light emitting diode (LED) that is adapted to emit light of a first wavelength from a patient facing extremity of the equipment module, a second LED that is adapted to emit light of a second wavelength from the patient facing extremity of the equipment module, and six photodetectors. The PPG device of the first aspect includes a mask that covers the patient facing extremity of the equipment module so that when the PPG device is applied to a patient the mask is situated between the patient and the patient facing extremity. The mask comprises a first window that registers with both the first LED and second LED and six second windows spaced from the first window and configured to that register with a respective one of the six photodetectors.

In the PPG device of the first aspect, the mask includes an aluminum foil layer on a patient facing side of the mask. Optionally, the PPG device of the first aspect may be non-enveloping when applied to the patient. Further optionally, the photodetector of the first aspect may include a photodiode.. Alternatively or additionally, the photodetector may include a ring of photodetectors that may circumscribe the first and second LED's.

It is contemplated by the present disclosure that the PPG device of the first aspect may further include a processor that may be adapted to control a drive current and an operating time of the second LED to achieve an elevated body tissue temperature of a patient to which the PPG device may be applied. If desired, the PPG device of the first aspect may further have a temperature sensor that may be adapted to detect the elevated body tissue temperature. As such, the processor of the first aspect may be adapted to control the drive current and the operating time of the second LED to achieve and maintain the detected elevated body tissue temperature at a specified value. Optionally, the first wavelength may be from a visible portion of an electromagnetic spectrum and the second wavelength may be from an infrared portion of the electromagnetic spectrum.

According to a second aspect of the present disclosure, a photoplethysmography (PPG) device may include a first light emitting diode (LED) that may be adapted to emit light of a first wavelength, a second LED that may be adapted to emit light of a second wavelength, and a photodetector. The PPG device of the second aspect may also include a processor that may be adapted to control a drive current and/or an operating time of the second LED to achieve an elevated localized body tissue temperature of a patient to which the PPG device may be applied.

In some embodiments, the PPG device of the second aspect may further include a temperature sensor that may be adapted to detect the elevated localized body tissue temperature. If desired, the processor of the second aspect may be adapted to control the drive current and/or the operating time of the second LED to achieve and maintain the elevated localized body tissue temperature at a specified value. With regard to the second aspect, the first wavelength may be from a visible portion of an electromagnetic spectrum and the second wavelength may be from an infrared portion of the electromagnetic spectrum.

According to a third aspect of the present disclosure, a method for conducting a photoplethsymographic (PPG) procedure may include registering a first window of a mask with both the first LED and second LED and registering each of the six second windows of the mask, that are spaced from the first window, with a respective one of the six photodetectors. Illuminating a tissue sample with radiation of a first wavelength and radiation of a second wavelength, trapping radiation in the tissue sample, detecting at least a portion of tissue-reflected first wavelength radiation and tissue-reflected second wavelength radiation, and analyzing the tissue-reflected first wavelength radiation and a detected non-reintroduced second wavelength radiation to determine a parameter of interest.

In some embodiments, the method of the third aspect may further include warming the tissue sample with radiation of the second wavelength prior to detecting the at least a portion of tissue-reflected first wavelength radiation and the tissue-reflected second wavelength radiation. Optionally, the method of the third aspect may also include warming the tissue sample with radiation of the second wavelength prior to illuminating the tissue sample with radiation of the first wavelength and radiation of the second wavelength.

According to a fourth aspect of the present disclosure, a method for conducting a photoplethsymographic (PPG) procedure may include warming a tissue sample with radiation of a second wavelength, illuminating the tissue sample with radiation of a first wavelength and radiation of the second wavelength, detecting tissue-reflected first wavelength radiation and tissue-reflected second wavelength radiation, and analyzing the tissue-reflected first wavelength radiation and the tissue-reflected second wavelength radiation to determine a parameter of interest.

The invention will now be further described by way of example with reference to the accompanying drawings, in which:
Fig. 1 is a perspective, schematic, exploded view of a PPG device including a mask and an equipment module having two light emitting diodes (LED's) and six photodetectors;
Fig. 2 is a side elevation view of the PPG device of Fig. 1 with the mask covering the patient facing side of the equipment module and showing the PPG device applied to a patient represented by the patient's skin S;
Fig. 3 is a view showing the PPG device applied to a patient;
Fig. 4 is a set of bar graphs schematically illustrating the effect of the PPG device embodiments described herein on the signal received at the photodetectors;
Figs. 5 and 6 are a cross sectional side elevation view and an end elevation view of an enveloping embodiment of the PPG device;
Figs. 7 and 8 are a schematic and a perspective view showing a PPG device having light emitting diodes, LED drivers, a photodetector, a temperature sensor, and a processor adapted to regulate drive current and/or operating time of one of the LED's;
Fig. 9 is a view similar to Fig. 7 showing a PPG device with three LED's, one of which is dedicated to warming the patient's skin; and
Figs. 10 and 11 are views similar to those of Figs. 7 and 8 showing an embodiment which includes the mask of the embodiment of Figs. 1, 2, 5 and 6 and the temperature sensor and processor of Figs. 7 and 8.

The present invention is defined by the appended claims.

In this specification and drawings, features similar to or the same as features already described may be identified by reference characters or numerals which are the same as or similar to those previously used. Similar elements may be identified by a common reference character or numeral, with suffixes being used to refer to specific occurrences of the element.

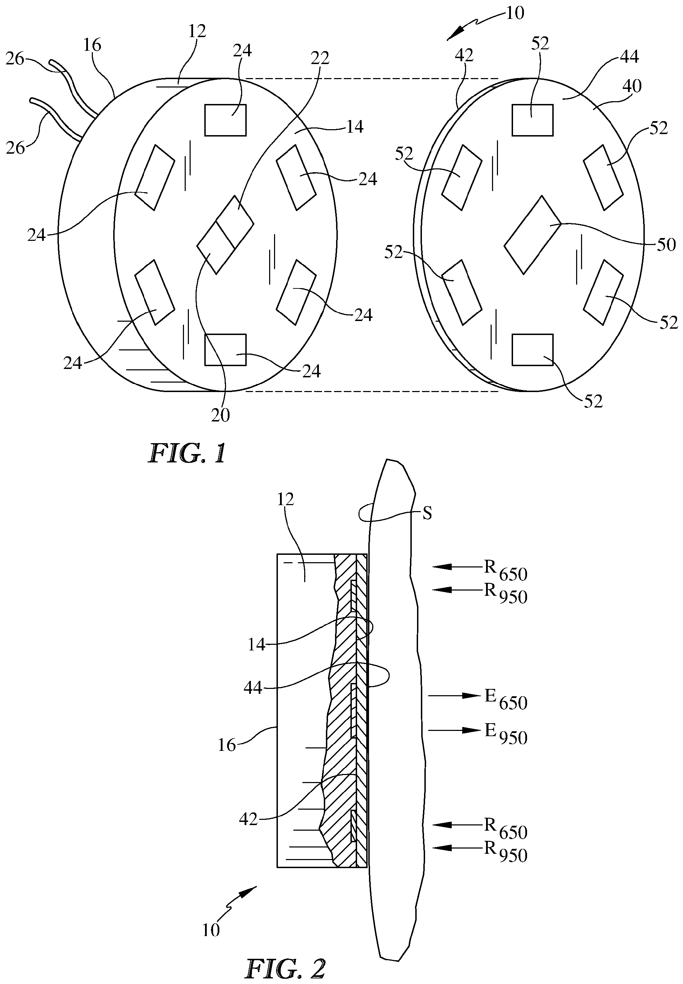

Referring to Fig. 1, a photoplethysmography (PPG) device 10 includes an equipment module 12. The equipment module 12 has a patient facing side or extremity 14 and an opposite side or extremity 16. When in use, the PPG device 10 is applied to the patient, for example by being secured to the patient, with the patient facing extremity 14 facing the patient and the opposite extremity 16 facing away from the patient. In Fig. 2 the patient is represented by a patch of skin S.

The equipment module 12 includes a first light emitting diode (LED) 20 adapted to emit light of a first wavelength λ₁ from the patient facing extremity 14 of the equipment module 12. In one embodiment the first wavelength is from the visible portion of the electromagnetic spectrum, for example about 650 nm which corresponds to visible red light. The equipment module 12 also includes a second light emitting diode 22 adapted to emit light of a second wavelength λ₂ from the patient facing extremity 14 of the equipment module 12. The second wavelength is from the infrared portion of the electromagnetic spectrum, for example about 950 nm. As is evident from the foregoing, the word "light," as used in this specification, is not limited to emissions in the visible portion of the electromagnetic spectrum.

The PPG device 10 of Fig. 1 also includes a driver, not illustrated but similar to drivers 60, 62 discussed below, for driving the LED's 20, 22.

The equipment module 12 also includes a photodetector 24 such as a photodiode. Photodiodes are used as the example of the photodetector in the balance of this specification. The illustrated embodiment includes an array of six photodiodes 24 arranged in a ring that circumscribes the first and second LED's 20, 22.

Wires 26 extend from opposite side 16 of the PPG device 10 to connect the device 10 to an electrical power source and, if necessary, to provide an information or data communication pathway. In other embodiments the device 10 may be powered by an on-board battery and/or the communication may be by wireless communication.

The PPG 10 device also includes a mask 40 having a module facing side 42 and a patient facing side 44. In one embodiment the mask 40 includes an aluminum foil layer that reflects radiation back into the tissue. Such a reflective mask 40 may reflect radiation across a broad spectrum of wavelengths or may be tailored to reflect radiation of only a selected wavelength band. In another embodiment, the mask 40 is an insulating mask which is not necessarily reflective.

Mask 40 includes a center window 50 and a set of six peripheral windows 52. As seen best in Fig. 2, the mask 40 covers the patient facing extremity 14 of the equipment module 12 so that when the PPG device 10 is applied to a patient, the mask 40 is situated between the patient and the patient facing extremity 14 of the equipment module 12. In addition, center window 50 registers with LED's 20, 22, and each peripheral window 52 registers with a respective one of the photodiodes 24.

Window 50 is transparent to the wavelengths of light emitted by LED's 20, 22. Taking 650 nm and 950 nm as an example, in one embodiment, the entire center window 50 is transparent to at least 650 nm and 950 nm light. In another embodiment, center window 50 has a first segment which overlies first LED 20 and is transparent to 650 nm light, and a second segment which overlies second LED 22 and is transparent to 950 nm light. Yet another embodiment includes two separate windows, one overlying the 650 nm first LED and transparent to 650 nm light, and the other overlying the 950 nm first LED and transparent to 950 nm light. Window or windows 50 may be openings, or may be a material having the requisite transparency.

In order for the photodiodes 24 to receive light reflected from the patient's tissue at both the first and second wavelengths, peripheral windows 52 are transparent to those wavelengths. Windows 52 may be openings, or may be a material having the requisite transparency.

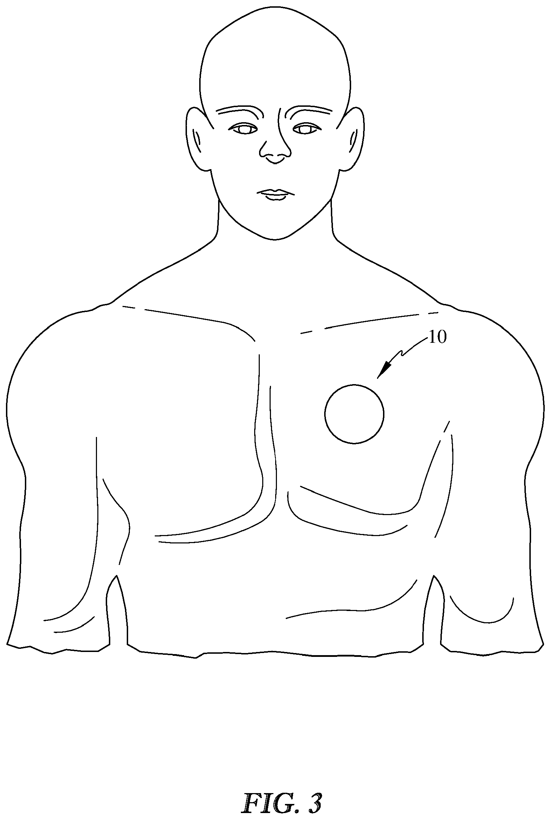

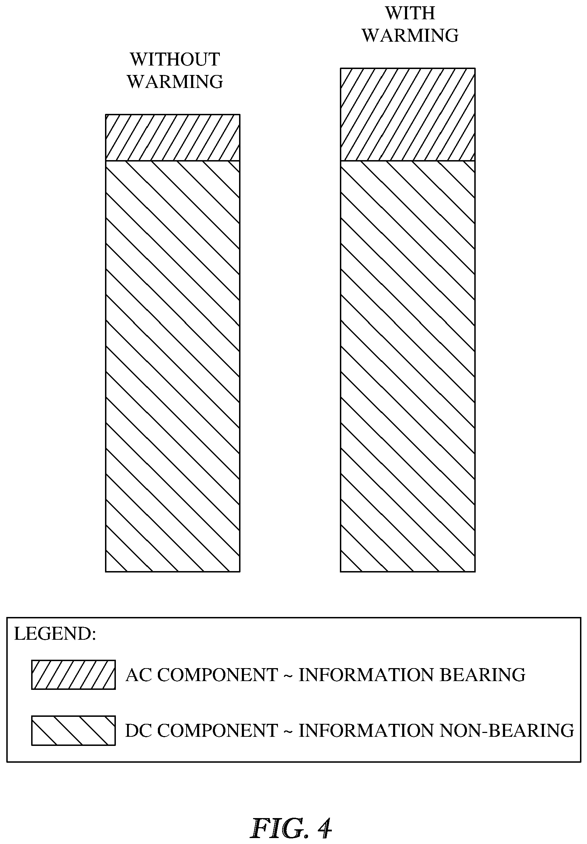

In operation, the device 10 is applied to the patient as seen best in Figs. 2 and 3. Fig. 3 shows the PPG device 10 applied to the patient's chest, however it may be applied elsewhere. Continuing to use the example of 650 and 950 nm light, the light emissions from first and second LED's enter the patient's tissue (arrows E₆₅₀, E₉₅₀). Light reflected back from the patient's tissue, R₆₅₀, R₉₅₀, arrives at the photodiodes 24 due to the transparency of peripheral windows 52 to the 650 and 950 wavelengths. The tissue-reflected radiation that does not pass through windows 52 (or through window 50) is trapped in the tissue by the mask 40. The trapped radiation, whether or not introduced by the 650 nm and 950 nm light sources, causes warming of the tissue above and beyond any warming that might occur if the mask 40 were not present. The pulsating arterial blood flow increases in response to the warmth. The tissue other than the pulsating arterial blood (e.g. flesh, bone) is largely or entirely unaffected by the warmth. Referring additionally to Fig. 4, the information bearing AC portion of the "warm" signal received at the photodetectors 24 is therefore amplified in comparison to the "cold" AC signal that would be obtained without the warming attributable to the mask 40. The "warm" AC signal is therefore larger than the "cold" AC signal, both in absolute terms and in proportion to the DC component of the signal. As a result the "warm" AC signal is easier to detect, and its information content is easier to extract than is the case with the "cold" AC signal.

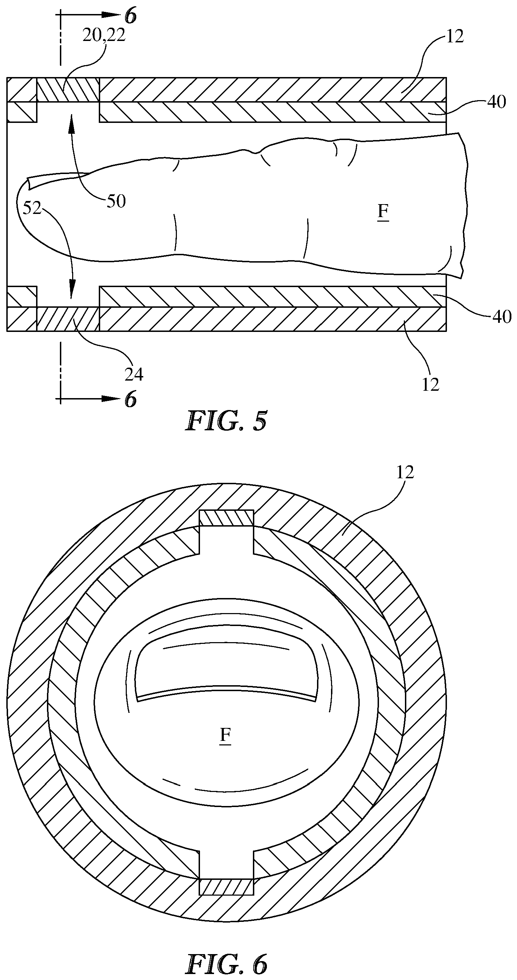

As seen in Fig. 2 and 3, the PPG device 10, as applied to a patient, is non-enveloping. In other words, the device 10 does not envelope a portion of the patient's anatomy. Figs. 5 and 6 show an alternative in which the PPG device envelopes or partially envelops a patient's finger F. The space between the patient and the PPG device is exaggerated to preserve the clarity of the illustration. Because the LED's 20, 22 and photodetector 24 are on opposite sides of the patient's finger, the device of Figs. 5 and 6 is a transmittance PPG device. The principles already described nevertheless apply. That is, mask 40 on module 12 traps radiation in the patient's finger F causing the tissue warming and beneficial effect on the AC component of the signal received at the photodetector 24 as already described.

A method for conducting a photoplethsymographic (PPG) procedure in accordance with the embodiments described above includes illuminating a tissue sample with radiation of a first wavelength and radiation of a second wavelength, and trapping radiation in the tissue sample. The method includes detecting at least a portion of tissue-reflected first wavelength radiation and tissue-reflected second wavelength radiation (i.e. the radiation that was not trapped in the tissue). The method also includes analyzing the detected first wavelength radiation and the detected second wavelength radiation to determine a parameter of interest. Such parameter of interest may include blood oxygen saturation, to name just one of many that will occur to those skilled in the art.

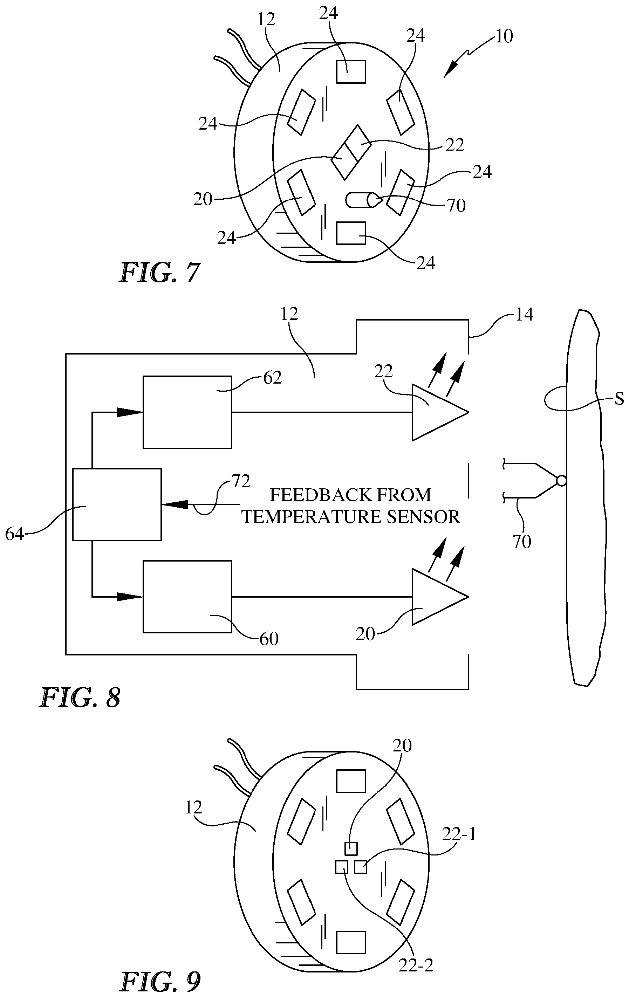

Referring to Figs. 7 and 8 a PPG device 10 includes a first LED 20 adapted to emit light of a first wavelength λ₁ (e.g. a visible wavelength such as 650 nm), a second LED 22 adapted to emit light of a second wavelength λ₂ (e.g. an infrared wavelength such as 950 nm) and a photodetector 24. The foregoing description of these components in connection with the PPG device 10 shown in Figs. 1, 2, 5 and 6 also applies to the device 10 of Figs. 7 and 8. The PPG device 10 also includes first and second LED drivers 60, 62 and a processor 64. A patient is represented by a patch of skin S. The space between the patient and the PPG device 10 is exaggerated to preserve the clarity of the illustration.

Processor 64 is adapted to control the drive current output from second LED driver 62 and/or the operating time of the second LED 22 in order to achieve an elevated localized body tissue temperature of the patient to which the PPG device 10 is applied. Elevated temperature means a temperature higher than that which would result if the drive current and/or LED operating time were not controlled in a way intended to achieve a tissue temperature high enough to boost the AC portion of the signal received at the photodetector 24 as seen in Fig. 4. For example the processor 64 may cause the driver 62 to supply more drive current to the second LED 22 than would be the case if the thermal effect on the tissue and the accompanying beneficial effect on the AC portion of the signal (FIG. 4) were not sought. Or, the processor 64 may cause the second LED 22 to illuminate the tissue for longer time intervals than would be necessary if the thermal effect on the tissue and the accompanying beneficial effect on the AC portion of the signal (FIG. 4) were not sought. Or, the processor 64 may command a combination of increased drive current and increased LED operating time.

The PPG may also include a temperature sensor adapted to detect the body tissue temperature. In Figs. 7 and 8 the temperature sensor is illustrated as a thermocouple 70. Feedback 72 from the temperature sensor 70 is used to control drive current and/or operating time of the second LED 22 to achieve and maintain the detected body tissue temperature at a specified value. One temperature believed to be satisfactory is about 40° C.

Fig. 9 shows an embodiment of the PPG device of Figs. 7 and 8 having a first LED 20 adapted to emit light of a first wavelength λ₁ (e.g. a visible wavelength such as 650 nm). The PPG device also includes an array of one or more second LED's 22-1, 22-2 all adapted to emit light of a second wavelength λ₂ (e.g. an infrared wavelength such as 950 nm). The second LED's 22-1, 22-2 provide illumination at a second (e.g. infrared) wavelength, just as in a conventional PPG device. The second LED's 22-1, 22-2 also help to achieve the elevated body tissue temperature. In one embodiment, several second LED's are provided in order to achieve the desired tissue warming effect and the attendant beneficial effect on the AC portion of the signal as shown in Fig. 4. In addition, the embodiment of Fig. 9 may include the temperature sensor of Figs. 7 and 8 for the purposes already described.

A method for conducting a photoplethsymographic (PPG) procedure in accordance with the embodiments described above includes warming a tissue sample with radiation of a second wavelength and illuminating the tissue sample with radiation of a first wavelength and radiation of the second wavelength. The method also includes detecting tissue-reflected first wavelength radiation and tissue-reflected second wavelength radiation. The method also includes analyzing the detected first wavelength radiation and the detected second wavelength radiation to determine a parameter of interest.

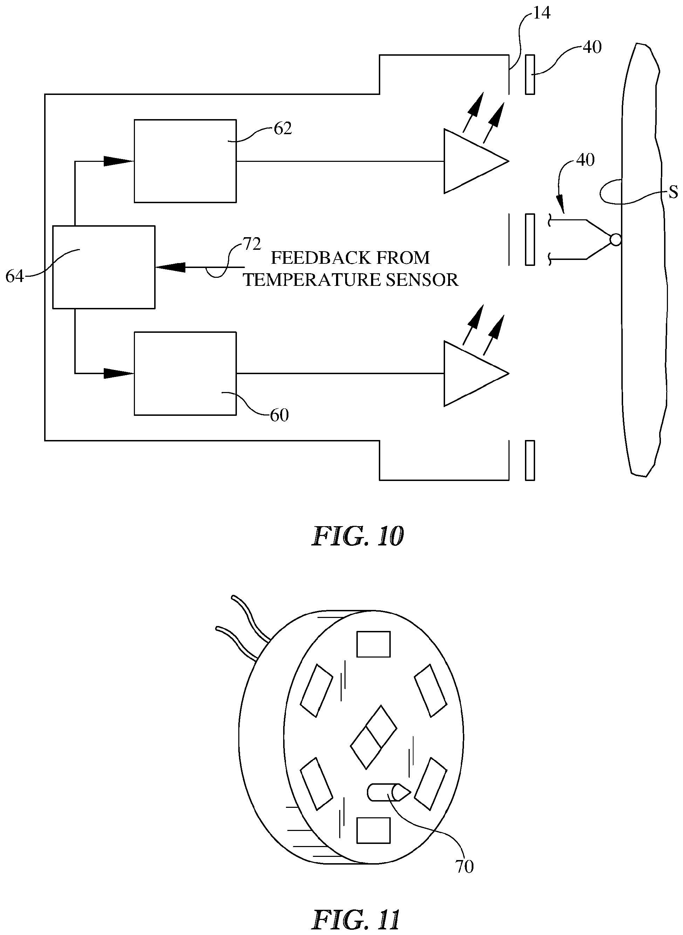

Figs. 10 and 11 show a PPG device which includes both a mask 40 as in Figs. 1, 2, 5 and 6, and a processor 64 adapted to control drive current and operating time of the second LED to achieve an elevated localized body tissue temperature of a patient to which the PPG device is applied as in Figs. 7 and 9. The embodiment of Figs. 10 and 11 combines the features and operational benefits of the embodiments of Figs. 1, 2, 5 and 6 with the features and operational benefits of the embodiment of Figs. 7 and 9.

A method for conducting a photoplethsymographic (PPG) procedure in accordance with the combined embodiments described above includes warming a tissue sample with radiation of a second wavelength and trapping heat energy in the tissue sample. The method also includes illuminating the tissue sample with radiation of a first wavelength and radiation of the second wavelength. The method also includes detecting at least a portion of tissue-reflected first wavelength radiation and at least a portion of tissue-reflected second wavelength radiation (i.e. the tissue reflected first and second wavelength radiation that was not trapped in the tissue). The method also includes analyzing the detected first wavelength radiation and the detected second wavelength radiation to determine a parameter of interest.

Although this disclosure refers to specific embodiments, it will be understood by those skilled in the art that various changes in form and detail may be made.

## Claims

1. A photoplethysmography, PPG, device (10) comprising:
an equipment module (12) which includes:
a first light emitting diode, LED, (20) adapted to emit light of a first wavelength from a patient facing extremity (14) of the equipment module (12);
a second LED (22) adapted to emit light of a second wavelength from the patient facing extremity (14) of the equipment module (12); and
six photodetectors (24); and
a mask (40) covering the patient facing extremity (14) of the equipment module (12) so that when the PPG device (10) is applied to a patient the mask (40) is situated between the patient and the patient facing extremity (14), **characterized in that** the mask comprises a first window that registers with both the first LED and the second LED and six second windows, each second window spaced from the first window and configured to register with a respective one of the six photodetectors and wherein the mask includes an aluminum foil layer on a patient facing side (44) of the mask.

2. The PPG device (10) of claim 1, wherein the PPG device (10) is non-enveloping when applied to the patient.

3. The PPG device (10) of any preceding claim, wherein the photodetectors (24) comprise a photodiode.

4. The PPG device (10) of claim 1, wherein the photodetectors (24) comprise
a ring of photodetectors (24) circumscribing the first and second LED's (20, 22).

5. The PPG device (10) of any preceding claim, further comprising a processor (64) adapted to control a drive current and an operating time of the second LED (22) to achieve an elevated body tissue temperature of a patient to which the PPG device (10) is applied.

6. The PPG device (10) of claim 5, further comprising a temperature sensor (70) adapted to detect the elevated body tissue temperature.

7. The PPG device (10) of claim 6, wherein the processor (64) is adapted to control the drive current and the operating time of the second LED (22) to achieve and maintain the detected elevated body tissue temperature at a specified value.

8. The PPG device (10) of any preceding claim, wherein the first wavelength is from a visible portion of an electromagnetic spectrum and the second wavelength is from an infrared portion of an electromagnetic spectrum.

9. A method for conducting a photoplethsymographic, PPG, procedure using the PPG device (10) of any preceding claim, the method comprising:
registering a first window of a mask, the mask including an aluminum foil layer on a patient facing side, with both the first LED and the second LED and registering each of the six second windows of the mask, that are spaced from the first window, with a respective one of the six photodetectors;
illuminating a tissue sample with radiation of a first wavelength and radiation of a second wavelength using the first and second LEDs
trapping radiation in the tissue sample by the mask;
detecting at least a portion of tissue-reflected first wavelength radiation and tissue-reflected second wavelength radiation using the photodetectors;
and
analyzing, by the PPG device (10), the tissue-reflected first wavelength radiation and a detected non-reintroduced second wavelength radiation to determine a parameter of interest.

10. The method of claim 9, further comprising warming the tissue sample with radiation of the second wavelength prior to detecting the at least a portion of tissue-reflected first wavelength radiation and the tissue-reflected second wavelength radiation.

11. The method of either claim 9 or claim 10, further comprising warming the tissue sample with radiation of the second wavelength prior to illuminating the tissue sample with radiation of the first wavelength and radiation of the second wavelength.

## Patentansprüche

1. Photoplethysmographie, PPG,
Vorrichtung (10), umfassend:
ein Gerätemodul (12), das einschließt:
eine erste lichtemittierende Diode, LED, (20), angepasst, um Licht einer ersten Wellenlänge von einer patientenseitigen Stirnseite (14) des Gerätemoduls (12) aus zu emittieren;
eine zweite LED (22), angepasst, um Licht einer zweiten Wellenlänge von der patientenseitigen Stirnseite (14) des Gerätemoduls (12) aus zu emittieren; und
sechs Photodetektoren (24); und
eine Maske (40), die die patientenseitige Stirnseite (14) des Gerätemoduls (12) abdeckt, so dass, wenn die PPG-Vorrichtung (10) an einen Patienten angelegt wird, die Maske (40) zwischen dem Patienten und der patientenseitigen Stirnseite (14) angeordnet ist, **dadurch gekennzeichnet, dass** die Maske ein erstes Fenster umfasst, das mit sowohl der ersten LED als auch der zweiten LED fluchtet, und sechs zweite Fenster, wobei jedes zweite Fenster von dem ersten Fenster beabstandet und angepasst ist, um mit einem jeweiligen der sechs Photodetektoren zu fluchten, und wobei die Maske eine Aluminiumfolienschicht auf einer patientenseitigen Seite (44) der Maske umfasst.

2. PPG-Vorrichtung (10) nach Anspruch 1, wobei die PPG-Vorrichtung (10) nicht umhüllend ist, wenn sie an den Patienten angelegt wird.

3. PPG-Vorrichtung (10) nach einem vorstehenden Anspruch, wobei die Photodetektoren (24) eine Photodiode umfassen.

4. PPG-Vorrichtung (10) nach Anspruch 1, wobei die Photodetektoren (24) einen Ring von Photodetektoren (24) umfassen, der die erste und die zweite LED (20, 22) umgibt.

5. PPG-Vorrichtung (10) nach einem vorstehenden Anspruch, weiter umfassend einen Prozessor (64), angepasst, um einen Treiberstrom und eine Betriebszeit der zweiten LED (22) zu steuern, um bei einem Patienten, an den die PPG-Vorrichtung (10) angelegt wird, eine erhöhte Körpergewebetemperatur zu erreichen.

6. PPG-Vorrichtung (10) nach Anspruch 5, weiter umfassend einen Temperatursensor (70), angepasst, um die erhöhte Körpergewebetemperatur zu detektieren.

7. PPG-Vorrichtung (10) nach Anspruch 6, wobei der Prozessor (64) angepasst ist, um den Treiberstrom und die Betriebszeit der zweiten LED (22) zu steuern, um die detektierte erhöhte Körpergewebetemperatur bei einem vorgegebenen Wert zu erreichen und aufrechtzuerhalten.

8. PPG-Vorrichtung (10) nach einem vorstehenden Anspruch, wobei die erste Wellenlänge aus einem sichtbaren Bereich des elektromagnetischen Spektrums ist und die zweite Wellenlänge aus einem Infrarotbereich des elektromagnetischen Spektrums ist.

9. Verfahren zum Durchführen einer photoplethysmographischen PPG-Prozedur unter Verwendung der PPG-Vorrichtung (10) nach einem vorstehenden Anspruch, wobei das Verfahren umfasst:
Fluchten eines ersten Fensters einer Maske, wobei die Maske eine Aluminiumfolienschicht auf einer patientenseitigen Seite einschließt, mit sowohl der ersten LED als auch der zweiten LED und Fluchten jedes der sechs zweiten Fenster der Maske, die von dem ersten Fenster beabstandet sind, mit einem jeweiligen der sechs Photodetektoren;
Beleuchten einer Gewebeprobe mit Strahlung einer ersten Wellenlänge und Strahlung einer zweiten Wellenlänge unter Verwendung der ersten und zweiten LEDs
Zurückhalten von Strahlung in der Gewebeprobe durch die Maske;
Detektieren mindestens eines Teils von vom Gewebe reflektierter Strahlung einer ersten Wellenlänge und von vom Gewebe reflektierter Strahlung einer zweiten Wellenlänge unter Verwendung der Photodetektoren; und
Analysieren, durch die PPG-Vorrichtung (10), der vom Gewebe reflektierten Strahlung einer ersten Wellenlänge und einer detektierten nicht wieder eingebrachten Strahlung einer zweiten Wellenlänge, um einen interessierenden Parameter zu bestimmen.

10. Verfahren nach Anspruch 9, weiter umfassend Erwärmen der Gewebeprobe mit Strahlung der zweiten Wellenlänge vor dem Detektieren des mindestens einen Teils von vom Gewebe reflektierter Strahlung einer ersten Wellenlänge und der vom Gewebe reflektierten Strahlung einer zweiten Wellenlänge.

11. Verfahren nach entweder Anspruch 9 oder Anspruch 10, weiter umfassend Erwärmen der Gewebeprobe mit Strahlung der zweiten Wellenlänge vor dem Beleuchten der Gewebeprobe mit Strahlung der ersten Wellenlänge und Strahlung der zweiten Wellenlänge.

## Revendications

1. Dispositif de photopléthysmographie, PPG, (10) comprenant :
un module d'équipement (12) qui inclut :
une première diode électroluminescente, DEL, (20) conçue pour émettre de la lumière d'une première longueur d'onde depuis une extrémité (14) du module d'équipement (12) tournée vers le patient ;
une deuxième DEL (22) conçue pour émettre de la lumière d'une deuxième longueur d'onde depuis l'extrémité (14) du module d'équipement (12) tournée vers le patient ; et six photodétecteurs (24) ; et
un masque (40) recouvrant l'extrémité (14) du module d'équipement (12) tournée vers le patient de telle sorte que, lorsque le dispositif PPG (10) est appliqué à un patient, le masque (40) est situé entre le patient et l'extrémité (14) tournée vers le patient, **caractérisé en ce que** le masque comprend une première fenêtre qui coïncide à la fois avec la première DEL et la deuxième DEL et six deuxièmes fenêtres, chaque deuxième fenêtre étant espacée de la première fenêtre et configurée pour coïncider avec un photodétecteur respectif des six photodétecteurs et dans lequel le masque inclut une couche de feuille d'aluminium sur un côté (44) du masque tourné vers le patient.

2. Dispositif PPG (10) selon la revendication 1, dans lequel le dispositif PPG (10) est non enveloppant lorsqu'il est appliqué au patient.

3. Dispositif PPG (10) selon une quelconque revendication précédente, dans lequel les photodétecteurs (24) comprennent une photodiode.

4. Dispositif PPG (10) selon la revendication 1, dans lequel les photodétecteurs (24) comprennent un anneau de photodétecteurs (24) entourant les première et deuxième DEL (20, 22).

5. Dispositif PPG (10) selon une quelconque revendication précédente, comprenant en outre un processeur (64) conçu pour commander un courant d'excitation et une durée de fonctionnement de la deuxième DEL (22) afin d'obtenir une température élevée du tissu corporel d'un patient auquel le dispositif PPG (10) est appliqué.

6. Dispositif PPG (10) selon la revendication 5, comprenant en outre un capteur de température (70) conçu pour détecter la température élevée du tissu corporel.

7. Dispositif PPG (10) selon la revendication 6, dans lequel le processeur (64) est conçu pour commander le courant d'excitation et la durée de fonctionnement de la deuxième DEL (22) afin d'obtenir et de maintenir la température élevée du tissu corporel détectée à une valeur spécifiée.

8. Dispositif PPG (10) selon une quelconque revendication précédente, dans lequel la première longueur d'onde provient d'une partie visible d'un spectre électromagnétique et la deuxième longueur d'onde provient d'une partie infrarouge d'un spectre électromagnétique.

9. Procédé de réalisation d'une procédure de photopléthysmographie, PPG, utilisant le dispositif PPG (10) selon une quelconque revendication précédente, le procédé comprenant :
le fait de faire coïncider une première fenêtre d'un masque, le masque incluant une couche de feuille d'aluminium sur un côté tourné vers le patient, avec à la fois la première DEL et la deuxième DEL et le fait de faire coïncider chacune des six deuxièmes fenêtres du masque, qui sont espacées de la première fenêtre, avec un photodétecteur respectif des six photodétecteurs ;
l'éclairage d'un échantillon de tissu avec un rayonnement d'une première longueur d'onde et un rayonnement d'une deuxième longueur d'onde en utilisant les première et deuxième DEL,
le piégeage du rayonnement dans l'échantillon de tissu par le masque ;
la détection d'au moins une partie du rayonnement de première longueur d'onde réfléchi par le tissu et du rayonnement de deuxième longueur d'onde réfléchi par le tissu en utilisant les photodétecteurs ; et
l'analyse, par le dispositif PPG (10), du rayonnement de première longueur d'onde réfléchi par le tissu et d'un rayonnement de deuxième longueur d'onde non réintroduit détecté afin de déterminer un paramètre d'intérêt.

10. Procédé selon la revendication 9, comprenant en outre le réchauffement de l'échantillon de tissu avec un rayonnement de la deuxième longueur d'onde avant de détecter l'au moins une partie de rayonnement de première longueur d'onde réfléchi par le tissu et le rayonnement de deuxième longueur d'onde réfléchi par le tissu.

11. Procédé selon la revendication 9 ou revendication 10, comprenant en outre le réchauffement de l'échantillon de tissu avec un rayonnement de la deuxième longueur d'onde avant l'éclairage de l'échantillon de tissu avec un rayonnement de la première longueur d'onde et un rayonnement de la deuxième longueur d'onde.
